Europäisches Patentamt

European Patent Office

Office européen des brevets

(11) Veröffentlichungsnummer: **0 077 779**
**B1**

(12) **EUROPÄISCHE PATENTSCHRIFT**

(45) Veröffentlichungstag der Patentschrift:
**20.11.86**

(51) Int. Cl.⁴: **A 61 M 5/28, A 61 J 1/06**

(21) Anmeldenummer: **82900616.2**

(22) Anmeldetag: **01.03.82**

(86) Internationale Anmeldenummer:
**PCT/CH 82/00030**

(87) Internationale Veröffentlichungsnummer:
**WO 82/03777 (11.11.82 Gazette 82/27)**

(54) **GEBRAUCHSFERTIGE EINWEG-INJEKTIONSPACKUNG.**

(30) Priorität: **29.04.81 CH 2767/81**

(43) Veröffentlichungstag der Anmeldung:
**04.05.83 Patentblatt 83/18**

(45) Bekanntmachung des Hinweises auf die Patenterteilung:
**20.11.86 Patentblatt 86/47**

(84) Benannte Vertragsstaaten:
**AT DE FR GB NL**

(56) Entgegenhaltungen:
**GB - A - 975 873**
**US - A - 2 744 527**
**US - A - 3 736 933**
**US - A - 3 757 981**
**US - A - 4 130 117**

(73) Patentinhaber: **HOLZNER, Günter, 8, Rue Montfalcon,
CH-1227 Carouge (CH)**

(72) Erfinder: **HOLZNER, Günter, 8, Rue Montfalcon,
CH-1227 Carouge (CH)**

ACTORUM AG

**Beschreibung**

Gegenstand der Erfindung ist die serienmässige Herstellung einer preiswerten, sterilisierbaren und gebrauchsfertigen Einweg-Injektionspackung. Bei dieser Packung ist die Injektionsflüssigkeit bereits eingefüllt und die Injektionskanüle befestigt. Die bisher verwendete Glasampulle zur Aufbewahrung der Injektionslösung sowie das Aufsetzen der Kanüle und das Einsaugen in die Injektionsspritze sind nicht mehr nötig.

Stand der Technik

Die derzeit für zahlreiche Injektionen verwendeten Einmalspritzen, meist aus Kunststoff oder Glas hergestellt, haben prinzipiell den gleichen Aufbau und die gleiche Funktion wie die seit langem bekannten, sterilisierbaren und oftmals verwendbaren Injektionsspritzen. Die Injektionslösung wird in einer eigenen Ampulle aufbewahrt die vor Gebrauch geöffnet wird um die Injektionslösung in die Spritze zu saugen.

Injektionskanüle und Spritze sind meist getrennt verpackt und werden vor Gebrauch zusammengesetzt. Dieses Verfahren erfordert einige Handgriffe die, falls nicht sachkundig ausgeführt, die Gefahr einer Infektion der Injektionslösung oder Kanüle in sich bergen.

Die gebrauchsfertige Injektionspackung, die Gegenstand dieser Erfindung ist, vermeidet jegliches Zusammensetzen und Füllen der Injektionsspritze. Sie ist sofort funktionsbereit und ausserdem billiger und einfacher in der Herstellung, da sie keine mechanisch bewegten Teile wie Kolben und Zylinderwand benötigt.

Die US-A-3 736 933 beschreibt bereits eine ähnliche Injektionspackung die aus einem verschlossenem Plastikschlauch besteht der an einem Ende mit einer Injektionskanüle versehen ist. Um das Ausfliessen der Injektionslösung vor Gebrauch zu verhindern befindet sich zwischen Injektionskanüle und dem gefüllten Plastikschlauch eine Schweissnaht mit mechanischer Sollbruchstelle. Diese bricht bei Fingerdruck auf die Aussenseite des Schlauches auf und gibt den Weg in die Injektionskanüle frei.

Bei praktischen Versuchen stellte sich jedoch heraus, dass es bei der Massenproduktion schwierig ist die Sollbruchstelle so zu gestalten dass sie immer bei gleichem Fingerdruck ordnungsgemäss aufbricht. Aus diesem Grunde hat sich das genannte System in der medizinischen Praxis nicht durchsetzen können.

Ebenso beschreibt die GB-A-975 873 ein Ventil für eine medizinische Injektionsspritze welches bei Überdruck öffnet.

Gegenstand der vorliegenden Erfindung ist die Trennung von Injektionslösung und Kanüle durch eine spezielle Schweissnaht, die ein gleichmässiges und einwandfreies Öffnen der Verbindung Injektionslösung-Kanüle unmittelbar vor der Injektion ermöglicht.

Die Erfindung

Zur Herstellung einer lagerfähigen, jedoch sofort gebrauchsfertigen Injektionspackung wird die Injektionslösung in einen flexiblen Plastikbeutel eingeschweisst. Dieser Plastikbeutel ist an einem Ende über einen kurzen Kanal in Verbindung mit der Injektionskanüle. Im Sinne der Erfindung wird dieser Verbindungskanal durch eine spezielle Schweissnaht verschlossen. Wird vor der Injektion mit den Fingern oder einer zangenartigen Vorrichtung ein entsprechender Druck auf die Aussenwand des Beutels ausgeübt so wird die Schweissnaht durchbrochen und die Injektionslösung gelangt in die Kanüle.

Die Injektion erfolgt durch weiteren Druck der Finger oder der zangenartigen Vorrichtung auf die Aussenseite des Plastikbeutels bis dieser vollständig platt zusammengedrückt und entleert ist.

Es ist äusserst wichtig, dass der Plastikbeutel auch bei längerer Lagerung dicht verschlossen bleibt um eine absolute Sterilität der eingeschlossenen Injektionslösung zu gewährleisten.

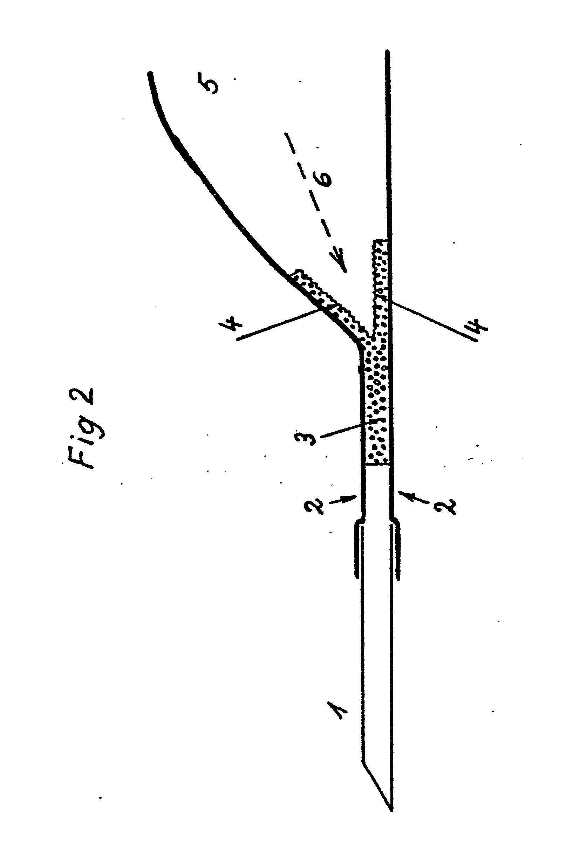

Der Verschluss kann im Sinne der Erfindung durch eine spezielle Schweissnaht erfolgen. Diese Schweissnaht befindet sich zwischen Auslassende des Plastikbeutels und Einlassöffnung der Injektionskanüle. Sie besteht aus einem dreiteiligen Folienverbund bei dem eine Schaumfolie oder Schaumschichte zwischen zwei festen Deckfolien eingeschweisst ist. Bei Druckbeanspruchung reisst die Schaumschichte in Längsrichtung auf, das heisst sie spaltet sich flächenmässig in zwei Hälften (siehe fig. 2).

Der Vorteil dieser flächenartigen Aufreissvorrichtung liegt darin, dass die zum Aufreissen nötige Kraft von der inneren Festigkeit der Schaumschichte abhängt welche in der Massenproduktion relativ leicht konstant gehalten werden kann. Ein zusätzlicher Vorteil besteht darin, dass die zwei aussenliegenden Deckfolien beim Aufbrechen der Verbindung vollständig intakt bleiben und dadurch jegliches Ausfliessen der Injektionslösung verhindern.

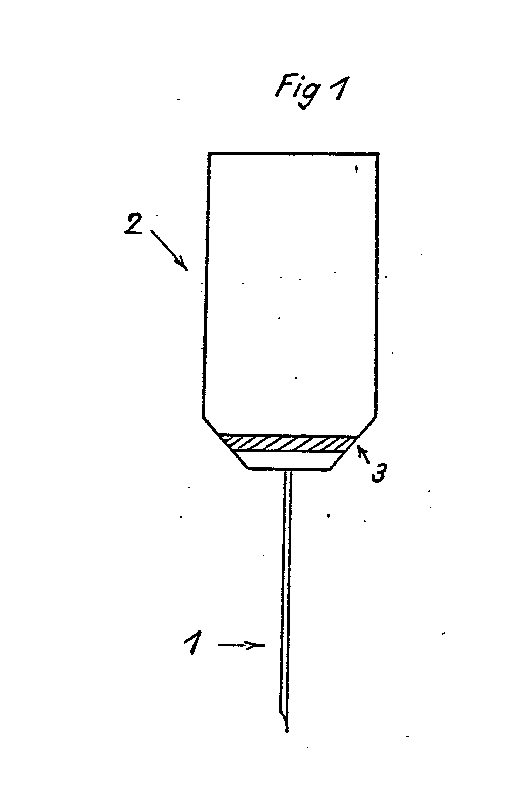

Die Schaumschichte kann aus einem polymeren Kunststoff wie Polyäthylen, Polypropylen, Polyamid, Polyvinylchlorid, Polystyrol oder einem anderen aufschäumbaren oder durch eine andere Behandlung porös gemachten plastischen Material bestehen. Aus Gründen der einfacheren Herstellung kann die Innenseite des Kunststoffbeutels ganz oder teilweise mit der beschriebenen Schaumfolie bedeckt sein. Die Ausführungsform der Erfindung ist in Fig. 1 schematisch dargestellt. Das Aufreissen der Schaumfolie ist in Fig. 2 ersichtlich.

Zeichenerklärung
Fig. 1
1   Injektionskanüle
2   Kunststoffbeutel
3   durch Überdruck aufbrechbare Schweissnaht mit zwischenliegender Schaumschichte

Fig. 2
1   Injektionskanüle
2   obere und untere Deckfolie des Kunststoffbeutels

3 eingeschweisste oder geklebte Schaumschichte

4 2 Teile der aufreissenden Schaumschichte

5 Injektionslösung

6 Weg der Injektionslösung bei Druck auf die Aussenseite des Kunststoffbeutels.

## Patentansprüche

1. Gebrauchsfertige Einweg-Injektionspackung bestehend aus einem verschlossenen, beim Ent-leeren vollständig zusammendrückbaren Kunst-stoffbeutel (2) der mit der Injektionslösung (5) gefüllt ist und an einem Ende in direkter Verbindung mit einer Injektionskanüle (1) steht, dadurch gekennzeichnet, dass die Verbindung zwischen Kunststoffbeutel (2) und Injektionskanüle (1) durch eine spezielle Schweissnaht gebildet wird welche aus einer Schaumschichte (3) besteht die zwischen zwei reissfeste Deckfolien eingeschweisst ist, und sich bei Druckbelastung in ihrer Längsrichtung aufspaltet ohne dass die beiden Deckfolien beschädigt werden.

2. Einweg-Injektionspackung nach Anspruch 1 dadurch gekennzeichnet, dass die aufspaltbare Schaumschichte (3) aus einem hochpolymeren Kunstoff besteht, eine Dicke von 0,005–1 mm aufweist und eine mehr oder weniger grosse Anzahl von kleinen Hohlräumen enthält die auf unterschiedliche Weise eingebracht werden können.

3. Einweg-Injektionspackung nach Anspruch 1 und 2 dadurch gekennzeichnet, dass die aufspaltbare Schaumschichte (3) nicht nur auf der Trennungsnaht zwischen Beutel und Kanüle liegt, sondern die Innenwand des Beutels ganz oder teilweise bedeckt.

## Claims

1. Injection syringe device, ready to use and disposable comprising a flexible plastic bag (2) which is filled with the fluid to be injected (5) and has mounted on one end an injection needle (1) characterised by a three layer breakable seal closing the end of the bag adjacent to the injection needle. Said three layer seal consists of a breakable foam layer (3) sealed between the rigid cover foils and is adapted to cleave the foam layer (3 and 4) without breaking the cover foils when a predetermined pressure is applied to the opposite walls of the plastic bag.

2. Disposable injection syringe device as claimed in claim 1, wherein the cleaving foam layer (3) consists of a polymeric plastic, has a thickness of 0,005–1 mm and contains variable numbers of pores which were introduced in different manners.

3. Disposable injection syringe device as claimed in claims 1 and 2 wherein the cleaving foam layer (3) covers not only the seal between bag and needle but also partially or entirely the inside of the plastic bag.

## Revendications

1. Emballage à injection prêt à l'emploi et jetable comprenant un sachet en plastique (2) fermé et complètement compressible qui est rempli avec la solution à injecter (5) et lié à une seringue à injection caractérisée par une soudure à 3 couches qui sépare la jonction entre sachet (2) et aiguille à injection (1). Cette soudure est formée par une couche de mousse (3) qui est soudée entre les deux feuilles de couverture du sachet (2) et se fend en direction longitudinale (4) sans que les deux feuilles de couverture soient endommagées.

2. Emballage à injection jetable selon revendication 1 caractérisée par une couche de mousse (3) en plastique ayant une épaisseur de 0,005 à 1 mm et contenant un nombre variable de petits espaces vides créés par des méthodes différentes.

3. Emballage à injection jetable selon revendications 1 et 2 caractérisé par le fait que la couche se fendant en mousse (3) ne se trouve pas seulement dans la soudure entre sachet et canule mais couvre aussi entièrement ou partiellement l'inté-rieur du sachet.

# Fig 1

Fig 2